# FASCICULE DE BREVET EUROPEEN

(11) **EP 1 552 820 B1**
(45) Date de publication et mention de la délivrance du brevet: **10.12.2008**
(21) Numéro de dépôt: 05300009.7
(22) Date de dépôt: 07.01.2005
(51) Int. Cl.: A61K 9/51, A61K 8/11, A61K 9/10

(54) **Dispersion aqueuse de nanocapsules à coeur huileux et son procédé de fabrication**
Wässrige Dispersion von Nanokapseln mit einem öligen Kern und deren Herstellungsverfahren
Aqueous dispersion of nanocapsules with an oily core and method of preparing it

(30) Priorité: 09.01.2004 FR 0450057
(43) Date de publication de la demande: 13.07.2005
(73) Titulaire: L'ORÉAL, 75008 Paris (FR)
(72) Inventeur: SIMONNET, Jean-Thierry, 94240, CACHAN (FR); RICHART, Pascal, 75013, PARIS (FR); BIATRY, Bruno, 94300, VINCENNES (FR)
(74) Mandataire: Le Coupanec, Pascale A.M.P.

(56) Documents cités:
- EP-A- 0 274 961
- EP-A- 1 025 901
- WO-A-99/04766
- WO-A-03/103822
- DE-A- 4 131 562
- RICHART P ET AL: "Nanocapsules: A ventor of choice for cosmetic lipophilic molecules" S.T.P. PHARMA PRATIQUES 2003 FRANCE, vol. 13, no. 1, 2003, pages 3-7, XP009038656 ISSN: 1157-1497
- "The Merck Index- Thirteenth Edition" 2001, , XP002302461 ISSN: 0911910-13-1 6088. Methylene Choloride: soluble in ca. 50 parts water * page 1082 *

## Description

La présente invention a pour objet principal une dispersion aqueuse de nanocapsules de type noyau/écorce, leur procédé de préparation et leur utilisation notamment dans le domaine de la cosmétique et/ou de la pharmacie.

Le domaine de l'invention concerne plus particulièrement celui de l'encapsulation d'au moins une huile, le cas échéant associée à au moins une matière active liposoluble dans un vecteur de type nanocapsule. De tels vecteurs sont particulièrement utiles dans les domaines de la cosmétique et de la pharmacie dans la mesure où ils garantissent une stabilité accrue à la matière active encapsulée et assure sa vectorisation efficace jusqu'à son site d'action, généralement l'épiderme.

De nombreuses techniques ont à ce jour été proposées pour préparer ce type de nanocapsules.

Par exemple, un certain nombre de nanocapsules sont aujourd'hui produites par une technique dite basculement de solvant. Cette technique est notamment illustrée dans le document EP 274 961. Elle implique la solubilisation de la matière à encapsuler et du polymère, devant constituer l'enveloppe de la nanocapsule, au sein d'un solvant. La formation des nanocapsules est initiée par addition, sous agitation, à cette solution d'un solvant annexe incapable de solubiliser le polymère et la matière à encapsuler mais en revanche miscible avec le premier solvant. Malheureusement, le taux d'encapsulation obtenu avec cette technique demeure insuffisant car il n'excède généralement pas 8 % en poids par rapport au poids de la dispersion.

Le document « Nanocapsules : un vecteur de choix pour les molécules lipophiles cosmétiques », de P. RICHART et J.T. SIMONNET décrit des nanocapsules présentant un noyau huileux entouré d'une coque polymérique le tout enrobé par un enrobage lamellaire permettant la vectorisation des molécules huileuses dudit noyau dans les structures cutanées, ainsi qu'un procédé de préparation basé sur l'émulsification spontanée d'une phase huileuse par son basculement via un solvant organique dans une phase aqueuse sous agitation lente.

La demande de brevet WO 01/64328 illustre une autre technique, qualifiée d'inversion de phase, qui implique l'application à l'émulsion considérée, de plusieurs cycles de montée et de descente en température pour provoquer cette inversion de phase. Cette technique y est notamment illustrée pour la préparation de particules de taille inférieure à 100 nm, avec un coeur huileux enrobé par une cire ou une lécithine, c'est-à-dire une écorce dont la température de fusion peut s'avérer inférieure à 20 °C. Ce type de paroi possède en outre l'inconvénient d'être très perméable, sensible aux tensioactifs et chimiquement instable car très sensible à l'oxydation.

Le brevet US 4 124 526 de Monsanto Company propose, pour sa part, une alternative à cette technique de basculement de solvant qui consiste à obtenir des particules par coacervation d'un sel d'un polymère poly-carboxylique, par acidification de la suspension à un pH compris entre 5 et 8. Ce procédé, sans solvant, conduit à des particules de tailles hétérogènes, généralement supérieures au micromètre et ayant une porosité élevée.

Une autre voie d'accès à ce type de nanocapsules repose sur la technique d'émulsification et implique la synthèse simultanée de l'écorce polymérique par réticulation des monomères correspondants.

Par exemple, le document FR 1 583 243 décrit un procédé de fabrication de microcapsules contenant des gouttelettes d'huile dont la taille varie de 0,1 à plusieurs microns et qui utilise des agents de réticulation chimiques. Cette technique est également illustrée dans le document WO 02/09862.

La demande WO 93/08908 utilise cette même technique pour préparer des nanocapsules dont l'écorce dérive de la réticulation de protéines.

La demande WO 02/051536 décrit quant à elle des suspensions de particules de taille comprise entre 70 nm et 5 µm, obtenues par réticulation de monomères, cette réticulation étant initiée par exposition de la suspension à une irradiation UV.

En fait, cette seconde voie d'accès a pour inconvénient majeur de nécessiter une étape de réticulation qui implique soit des espèces chimiques de type agent réticulant qui peuvent poser problème en terme d'innocuité, soit un stimuli externe de type irradiation UV qui peut être préjudiciable au niveau des actifs biologiques si ceux-ci s'avèrent sensibles aux UV.

Enfin, la demande WO 99/43426 des Laboratoires Sérobiologiques décrit des particules lipidiques solides entre 25 et 85 °C, ayant une taille comprise entre 10 nm et 5 mm, obtenues par émulsification et sans solvant. Toutefois, les particules décrites dans ce document sont des particules nécessairement constituées d'au moins un lipide solide de type cire et sont sans enrobage polymérique solide, c'est-à-dire différents de nanocapsules. Introduites dans une composition contenant des tensioactifs, ces particules manifestent une stabilité limitée en raison de la solubilisation partielle des cires par les tensioactifs, induisant ainsi la libération des actifs huileux encapsulés.

D'une manière générale, les procédés décrits ci-dessus sont en outre dépendants de la nature des solvants utilisés, qui doivent être miscibles à l'eau. Ceci implique une limitation importante dans le choix des polymères qui forment l'écorce de la nanocapsule, par défmition non solubles dans l'eau et lipophiles. Enfin, ces procédés nécessitent généralement des volumes de solvant (miscible à l'eau) et d'eau très importants qu'il faut ensuite évaporer, pour n'obtenir au final qu'une dispersion contenant au plus 8 % en poids en taux d'encapsulation.

En conséquence, les dispersions de nanocapsules actuellement disponibles ne s'avèrent pas totalement satisfaisantes dans la mesure où elles ne permettent pas de donner simultanément satisfaction en terme de taille, parfois d'innocuité, de stabilité et de polyvalence au niveau de la nature de la matière active à encapsuler.

Qui plus est, la teneur en matière active encapsulée n'excède généralement pas 8 % avec ce type de nanocapsules. Or, pour augmenter les performances des molécules encapsulées, il serait nécessaire de posséder un taux d'encapsulation, en tout état de cause, supérieur à 8 % et en particulier supérieur à 10 %.

La présente invention a précisément pour objectif de proposer des nanocapsules permettant de donner satisfaction en ces termes, c'est-à-dire appropriées à l'encapsulation en forte proportion d'une grande variété d'huiles et/ou d'actifs liposolubles, pouvant posséder avantageusement une gamme de taille allant de 40 nm à 150 nm, compatibles avec l'utilisation d'un très grand nombre de polymères et dont la préparation nécessite des quantités de solvant moindres que celles des procédés conventionnels.

La présente invention concerne, selon un de ses aspects, une dispersion aqueuse de nanocapsules de type noyau/écorce, dans lesquelles :
- le noyau comprend au moins une huile,
- l'écorce recouvrant le noyau est de nature polymérique, non réticulée, non hydrosoluble et non soluble dans l'huile dudit noyau,
lesdites nanocapsules présentant une taille moyenne inférieure ou égale à 500 nm et possèdant un taux d'encapsulation d'au moins 10% en poids exprimé en poids de matière(s) encapsulée(s) par rapport au poids total de ladite dispersion.

La présente invention concerne, selon un autre de ses aspects, un procédé de préparation d'une dispersion aqueuse de nanocapsules de type noyau/écorce dont le noyau comprend au moins une huile et l'écorce recouvrant le noyau est de nature polymérique non hydrosoluble et non soluble dans la ou les huile(s) dudit noyau, comprenant les étapes consistant à :
a) préparer un milieu organique liquide monophasique comprenant au moins :
   - un solvant organique, présentant une température d'ébullition inférieure ou égale à 100 °C,
   - un polymère soluble dans ledit milieu solvant,
   - une huile, contenant le cas échéant au moins une matière active liposoluble, et
   - un tensioactif non ionique,
b) préparer un milieu aqueux contenant au moins un tensioactif non ionique et le cas échéant un tensioactif ionique,
c) disperser le milieu organique (a) dans le milieu aqueux (b) de manière à obtenir une pré-émulsion,
d) soumettre la pré-émulsion obtenue en (c) à une homogénéisation de manière à obtenir la formation d'une dispersion de nanocapsules de taille moyenne inférieure ou égale à 500 nm,
e) évaporer le solvant organique et le cas échéant une partie de l'eau, la dispersion aqueuse obtenue à l'issue de l'étape e) présente un taux d'encapsulation supérieur ou égal à 10% en poids et
f) récupérer ladite dispersion aqueuse de nanocapsules ainsi obtenue.

Le procédé selon l'invention est avantageux à plusieurs titres.

Tout d'abord, comme il ressort de ce qui suit, il s'avère approprié à l'encapsulation d'une grande diversité d'huiles végétales, animales ou synthétique.

Les nanocapsules obtenues selon ce procédé peuvent posséder une taille moyenne inférieure à 150 nm, sans que ce soit préjudiciable au taux d'encapsulation qui demeure bien supérieur à 10 % en poids par rapport au poids total de la dispersion aqueuse.

Dans la mesure où il met en oeuvre des polymères pré-formés, il permet de s'affranchir efficacement du problème d'innocuité plus particulièrement posé par l'utilisation de monomères qui implique celle d'au moins un agent réticulant.

Enfin, le procédé de l'invention est avantageusement exempt des deux contraintes posées par la nature des solvants et par leur volume d'utilisation avec les procédés discutés ci-dessus.

### Nanocapsules :

Au sens de la présente invention, le terme "nanocapsules" désigne une structure de type noyau-écorce, c'est-à-dire une structure constituée d'un noyau formant ou contenant la matière à encapsuler, lequel noyau est enveloppé par une écorce protectrice continue et insoluble dans l'eau. En d'autres termes, il s'agit selon l'invention de nanocapsules à noyau lipidique entouré d'une écorce de nature polymérique.

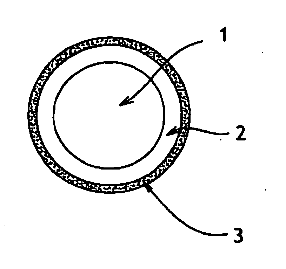

Les nanocapsules selon l'invention peuvent par exemple être telles que représentées schématiquement en figure 1.

Les nanocapsules représentées en figure 1 comprenant un noyau (1), comprenant au moins une huile, une écorce (2), de nature polymérique, et le cas échéant une phase lamellaire externe (3).

Les nanocapsules selon l'invention se distinguent notamment des "nanosphères" constituées d'une matrice polymérique poreuse dans laquelle la matière active est absorbée et/ou adsorbée.

Les nanocapsules selon l'invention possèdent avantageusement une taille inférieure ou égale à 500 nm, plus particulièrement inférieure ou égale à 250 nm. Selon un mode de réalisation particulier, elles possèdent une taille inférieure ou égale à 150 nm, voire une taille inférieure ou égale à 125 nm, notamment inférieure ou égale à 100 nm, et en particulier inférieure ou égale à 80 nm. Cette taille est exprimée en taille moyenne en intensité fournie par un granulomètre Brookhaven type BI 90 plus, dont le principe de mesure repose sur la diffusion quasi-élastique de la lumière (QELS).

Comme précisé précédemment, la dispersion selon l'invention possède un taux d'encapsulation significativement supérieur à celui possédé par les dispersions de nanocapsules conventionnelles telles que celles discutées précédemment.

Ce taux d'encapsulation exprimé en poids de matière(s) encapsulée(s) est supérieur ou égal à 10 % en poids exprimé en poids de matière(s) encapsulée(s) par rapport au poids total de ladite dispersion, et notamment supérieur ou égal à 12,5 % en poids, voire supérieur ou égal à 15 % en poids, et plus particulièrement supérieur ou égal à 17,5 % en poids par rapport au poids total de la dispersion.

L'écorce des nanocapsules est de nature polymérique, non réticulée, non hydrosoluble et non soluble dans l'huile du noyau.

D'une manière générale, tous les polymères, d'origine naturelle ou synthétique, solubles dans un solvant non miscible à l'eau et notamment ceux ayant un point de fusion inférieur au point d'ébullition de l'eau à la pression atmosphérique (100 °C), peuvent être utilisés selon la présente invention.

Ces polymères peuvent être biodégradables, comme par exemple les polyesters, ou non.

A titre illustratif des polymères convenant à l'invention, on peut notamment citer :
- les polymères de cyanoacrylate d'alkyle en C₂-C₁₂ et, en particulier, en C₂-C₆ avec le radical alkyle pouvant en particulier choisi parmi les radicaux éthyle, n-butyle, hexyle, isobutyle et isohexyle,
- les polymères formés par les poly-L-lactides, les poly-DL-lactides, les polyglycolides et les copolymères correspondants, tels que les copoly(DL-lactides et glycolides), copoly(glycolides et caprolactones) et similaires,
- les polycaprolactones, telles que les polycaprolactones ayant un point de fusion variant de 40 à 70 °C et de poids moléculaire compris entre 2000 et 100000, comme par exemple celles commercialisées par la société Solvay sous les références commerciales CAPA 6806 (PM de 80000), CAPA 6100 (PM de 10000), CAPA 6506 (PM 50000), CAPA 6250 (PM de 25000), CAPA 2803 (PM de 8000), CAPA 2403 D (PM de 4000),
- les polymères de l'acide 3-hydroxy butyrique,
- les copolymères de chlorure de vinyle et d'acétate de vinyle, par exemple ceux commercialisés sous la dénomination RHODOPAS AX 8515® par la société RHONE POULENC,
- les copolymères d'acide et d'ester méthacrylique, notamment d'acide méthacrylique et d'ester d'acide méthacrylique, par exemple ceux commercialisés sous la dénomination EUDRAGIT L 100® par la société ROHM PHARMA,
- l'acéto-phtalate de polyvinyle,
- l'acéto-phtalate de cellulose,
- le copolymère polyvinylpyrrolidone-acétate de vinyle,
- les polyéthylènevinylacétates,
- les polyacrylonitriles,
- les polyacrylamides,
- les polyéthylèneglycols,
- les poly-(méthacrylate d'hydroxyalkyle en Ci à C₄) et en particulier les poly-(méthacrylate d'hydroxyéthyle),
- les dérivés de cellulose tels que les esters de cellulose et d'au moins un acide carboxylique en Ci à C₄, notamment des esters de cellulose mixtes de deux types d'acides carboxyliques,
- le polystyrène et les copolymères de styrène et d'anhydride maléique, les copolymères de styrène et d'acide acrylique, les terpolymères séquencés styrène éthylène/butylène-styrène, les terpolymères séquencés styrène-éthylène/propylène-styrène,
- les oligomères styrène alkylalcool,
- les terpolymères d'éthylène, d'acétate de vinyle et d'anhydride maléique,
- les polyamides,
- les polyéthylènes,
- les polypropylènes,
- les organopolysiloxanes dont les polydiméthylsiloxanes,
- les poly (alkylène adipate) qui englobe à la fois les homopolymères d'acide adipique et d'un alcane-diol et les copolymères de type poly(ester éther), linéaires ou ramifiés, obtenus à partir d'acide adipique et d'un ou de plusieurs alcane-diols et/ou éthers-diols et/ou triols ; les alcane-diols utilisés pour la préparation desdits poly(alkylène adipate) peuvent être des alcane-diols en C₂-C₆ à chaîne linéaire ou ramifiée choisis parmi l'éthylèneglycol, le propylèneglycol, le 1,3-propanediol, le 1,4-butanediol, le 1,5-pentanediol, le 1,6-hexanediol et le néopentylglycol. Les éther-diols sont des di-, tri- ou tétra-(alkylène en C₂-C₄)-glycols tels que le diéthylèneglycol, le triéthylèneglycol, le tétraéthylèneglycol, le dipropylèneglycol, le tripropylèneglycol, le tétrapropylèneglycol, le dibutylèneglycol, le tributylèneglycol ou le tétrabutylèneglycol ; à titre d'exemple, on peut plus particulièrement citer les Fomrez® commercialisés par la société Witco et les Poly(éthylène) adipate de la société Scientific Polymer Products ; on peut également citer à titre d'exemple, l'Eastar Bio® de Eastman Chemical (poly (tetraméthylène Adipate co téréphtalate) et l'Ecoflex F BX 7011® de BASF ( terpolymère Butanediol 1,4 acide terephtalique et acide adipique),
- les polyesters polyol obtenus par polycondensation d'un acide dicarboxylique aliphatique avec au moins deux alcane-diols ou avec au moins un alcane-diol et au moins un hydroxyalkyl alcane-diol avec l'acide dicarboxylique aliphatique pouvant être l'acide adipique (acide hexane-1,6-dioïque) ; de tels polymères sont notamment décrits dans le document FR 2 836 381,
- les polymères siliconés de polysilsesquioxane, notamment un polyalkylsilsesquioxane de formule : (R-Si03/2)ₓₒ dans laquelle R représente un radical hydrocarboné, saturé ou insaturé, linéaire, ramifié ou cyclique; par exemple du type -CₙH₂ₙ₊₁, avec n étant un entier allant de 1 à 20, notamment un radical méthyle, éthyle, propyle, butyle, pentyle, hexyle, heptyle, octyle, nonyle, décyle, dodécyle, tridécyle, tétradécyle, hexadécyle, octadécyle et eicosyle; ou encore un groupe aryle, notamment phényle ou tolyle; un groupe cycloalkyle, notamment cyclobutyle, cyclopentyle ou cyclohexyle; un groupe alcényle, notamment vinyle ou allyle; un groupe aralkyle, notamment 2-phényléthyle ou benzyle; R pouvant également comprendre un ou plusieurs atomes d'halogène, notamment de fluor ou de chlore; de préférence R est un radical méthyle, éthyle, propyle ou phényle;et x est le nombre de motifs, et peut être compris entre 1 et 10, notamment 1 à 4 ; à titre d'exemple, on peut citer la Belsil PMS MK® de Wacker ou la Resin KR 220® de Shin Etsu,
- les polyesters dendritiques à fonction hydroxyle terminale notamment ceux décrits dans le document FR 2 790 405,
- les polymères hydrodipersibles mais néanmoins solubles dans des solvants non miscibles à l'eau comme par exemple : les polyesters, poly(ester amides), polyuréthannes et copolymères vinyliques portant des fonctions acide carboxylique et/ou sulfonique et en particulier ceux décrits dans le document FR 2 787 729,
- les copolymères blocs insolubles dans l'eau à température ambiante et solides à température ambiante, ayant au moins un bloc d'un des polymères précédents, et
- leur mélanges.

Ces polymères ou copolymères peuvent posséder un poids moléculaire en poids compris entre 1000 et 500 000 et en particulier entre 1500 et 100 000.

Conviennent tout particulièrement à l'invention, les poly(alkylène adipate), les organo polysiloxanes, les polycaprolactones, l'acétophtalate de cellulose, l'acétobutylate de cellulose, les esters de cellulose, le polystyrène, et ses dérivés.

Bien entendu, l'homme du métier est à même, de par ses connaissances, d'ajuster le poids moléculaire du polymère sélectionné vis-à-vis de sa concentration dans le solvant afin d'avoir une viscosité du mélange compatible avec une émulsification satisfaisante.

### Matière active susceptible d'être encapsulée :

Au sens de la présente invention, le terme « huile » désigne toutes les substances huileuses, liquides à partir de 40 °C, naturelles, végétales ou animales, ou synthétiques ayant ou non une ou plusieurs activités biologiques avérées et insolubles dans l'eau (moins de 2 % en poids à température ambiante).

A titre illustratif et non limitatif de ces huiles, on peut notamment citer les huiles végétales riches en insaturations comme l'huile de bourrache, les huiles de poisson, les filtres solaires, les vitamines E, F, K, leurs esters, et leurs mélanges.

Peuvent également être encapsulés une ou plusieurs matières actives liposolubles qui seront solubilisées dans une huile solvante. Selon cette variante, c'est le mélange d'au moins deux composantes voire plus qui est encapsulé. A titre d'exemple de ces principes actifs liposolubles, on peut citer les vitamines telles que la vitamine A (rétinol), la vitamine D, les carotènes, le β carotène, l'acide salicylique et leurs dérivés. Par exemple, peuvent être utilisés à titre de dérivés de l'acide salicylique ceux décrits dans les documents FR-A-2 581 542, EP-A-378 936 et EP-A-570230 et en particulier les acides noctanoyl-5-salicylique, n-décanoyl-5-salicylique, n-dodécanoyl-5-salicylique, noctyl-5-salicylique, n-heptyloxy-5-salicylique et n-heptyloxy-4-salicylique.

Par ailleurs, peuvent également être encapsulées, toutes molécules à effet thérapeutique utilisées dans le domaine pharmaceutique dans la mesure où elles présentent un caractère huileux et/ou une solubilité suffisante dans l'huile considérée. Il peut ainsi s'agir d'anti-inflammatoires, stéroïdiens ou non, d'antifongiques, d'antibactériens, d'antibiotiques, d'antimitotiques, d'anesthésiques, d'analgésiques, d'antiseptiques, d'antiviraux.

Mais il peut également s'agir de composés liposolubles actifs dans d'autres domaines, comme par exemple des pesticides ou des herbicides.

Bien entendu, le taux en huile(s) et le cas échéant en matière(s) active(s) liposoluble(s) (huile ou huile+actif) solubilisé dans le solvant, est fonction de la solubilité de ceux-ci.

D'une manière générale, il est ajusté de manière à ce que le taux d'encapsulation puisse représenter plus de 10 % et en particulier de 10 à 40 % du poids total de la dispersion aqueuse.

Comme précisé précédemment, les nanocapsules selon l'invention peuvent être préparées par le procédé conforme à l'invention.

Ce procédé selon l'invention implique notamment, successivement
- la solubilisation de l'huile, du ou des polymère(s) et le cas échéant de la matière active à encapsuler dans un milieu solvant organique, généralement non aqueux,
- la formation d'une pré-émulsion « milieu solvant organique dans un milieu aqueux » par dispersion de ce milieu organique dans un milieu aqueux, et
- l'homogénéisation généralement par haute pression de la pré-émulsion formée de manière à former une dispersion aqueuse de nanocapsules.

Le milieu solvant organique est ensuite éliminé, généralement par évaporation sous pression réduite, voire une partie de l'eau, de manière à obtenir une dispersion de nanocapsules à la concentration souhaitée.

Selon un mode de réalisation particulier, le rapport pondéral huile/polymère mis en oeuvre dans le milieu solvant organique est inférieur ou égal à 30/1 et notamment varie de 1/25 à 25/1.

### Milieu solvant organique :

Les milieux solvants organiques considérés selon l'invention sont non miscibles à l'eau, au moins en partie. Plus précisément, leur solubilité dans l'eau doit être inférieure à 10 % en poids dans l'eau à température ambiante. Leur point d'ébullition est nécessairement inférieur à celui de l'eau, généralement 100 °C à pression atmosphérique. A titre d'exemple de solvant convenant à l'invention, on peut en particulier citer l'acétate d'éthyle, l'acétate de butyle, le dichlorométhane, le cyclohexane, l'heptane, le chloro-1-butane, le chloroforme, le formate d'éthyle et leurs mélanges.

La proportion en milieu solvant organique peut être ajustée de manière à constituer de 5 et 70 % en poids du poids total de la pré-émulsion obtenue à l'étape c).

### Tensioactifs :

Afin de faciliter l'émulsification du milieu solvant organique incorporant l'huile et le cas échéant la matière active à encapsuler, et de maîtriser la stabilité de l'émulsion correspondante, il est généralement souhaitable d'utiliser au moins tensioactif, notamment un tensioactif non ionique.

Cet agent tensioactif ou ce mélange de tensioactifs est généralement présent de 0,05 à 25 % et notamment de 1 à 20 % en poids par rapport au poids du milieu solvant organique à disperser. Sa valeur HLB (Balance Hydrophile-Lipophile) est généralement ajustée pour être favorable à la formation des émulsions de type huile dans eau.

Conviennent notamment à l'invention, les agents tensioactifs non ioniques suivants :
- les alkylester ou éther de glycérol ou de polyglygérol constitués de 1 à 10 « motif » glycérol et d'au moins une chaîne alkyle (acide pour les esters et alcool pour les éthers) ayant de 12 à 22 atomes de carbone. Elle peut être saturée ou insaturée et ramifiée ou non. A titre d'exemple, on peut citer le Nikkol DGMS® (monostéarate de diglycérol), le Nikkol decaglyn 21S® (di isostéarate de décaglycérol), l'hexadécyléther de triglycérol,
- les esters mixtes d'acides gras ou d'alcool gras, d'acide carboxylique et de glycéryl choisis par exemple parmi les esters mixtes d'acide gras ou d'alcool gras en C₈-C₂₂ et d'alpha-hydroxyacide et/ou d'acide succinique avec la glycérine et leurs mélanges. A titre d'exemple, on peut citer l'ester mixte de glycérine et du mélange d'acides citrique, lactique, linoléique et oléique (nom CTFA : Glyceryl citrate/lactate/linoleate/oleate) commercialisé par la société Hüls sous la dénomination Imwitor 375® ; l'ester mixte d'acide succinique et d'alcool isostéarylique avec la glycérine (nom CTFA : Isostéaryl diglycéryl succinate) commercialisé par la société Hüls sous la dénomination Imwitor 780 K® ; l'ester mixte d'acide citrique et d'acide stéarique avec la glycérine (nom CTFA : Glycéryl stéarate citrate) commercialisé par la société Hüls sous la dénomination Imwitor 370® ; l'ester mixte d'acide lactique et d'acide stéarique avec la glycérine (nom CTFA : Glyceryl stearate lactate) commercialisé par la société Danisco sous la dénomination Lactodan B30® ou Rylo LA30®,
- les éthers gras éthoxylés ou esters gras éthoxylés comprenant de 2 à 50 unités d'oxyde d'éthylène et au moins une chaîne alkyle ( acide pour les esters et alcool pour les éthers) ayant de 12 à 22 atomes de carbone. Elle peut être saturée ou insaturée et ramifiée ou non. A titre d'exemple, on citera la série des Brij® ( alcools gras éthoxylés) commercialisée par la société Uniqema, la série des Myrj® (stéarates éthoxylés) commercialisée par la société Uniqema et l'isostéarate de PEG400 également commercialisé par Uniqema,
- les esters gras de sorbitan, oxyéthylénés ou non. Ils comprennent au moins un motif sorbitan et dans le cas où ils sont oxyéthylénés, de 2 à 50 motifs d'oxyde d'éthylène, et au moins une chaîne alkyle (acide gras) ayant de 12 à 22 atomes de carbone. Elle peut être saturée ou insaturée et ramifiée ou non. A titre d'exemple, on citera les séries des Span® (esters de sorbitan) et des Tween® (esters de sorbitan oxyéthylénés) commercialisées par Uniqema,
- les esters gras de sucre ou éthers gras de sucre. Le tensioactif utilisé est par exemple choisi parmi les esters d'acide gras en C₈ à C₂₂ de sucrose, maltose, glucose et fructose, les esters d'acide gras en C₁₄ à C₂₂ et de méthylglucose, les alkylpolyglucosides et leurs mélanges. La ou les chaînes alkyles peuvent être saturée ou insaturée et ramifiée ou non.

Les acides gras en C₈-C₂₂ ou en C₁₄-C₂₂ formant le motif gras des esters utilisables dans la composition de l'invention comportent au moins une chaîne alkyle linéaire saturée ou non saturée, comportant respectivement de 8 à 22 ou de 14 à 22 atomes de carbone. Le motif gras des esters peut être notamment choisi parmi les stéarates, béhénates, arachidonates, palmitates, myristates, laurates, caprates et leurs mélanges. On utilise notamment des stéarates.

On peut citer, à titre d'exemple d'esters ou de mélanges d'esters d'acide gras et de sucrose, de maltose, de glucose ou de fructose, le monostéarate de sucrose, le distéarate de sucrose, le tristéarate de sucrose et leurs mélanges, tels que les produits commercialisés par la société Croda sous la dénomination Crodesta® F50, F70, F110, F160 ayant respectivement un HLB (Balance Hydrophile Lipophile) de 5, 7, 11 et 16 ; et à titre d'exemple d'esters ou de mélanges d'esters d'acide gras et de méthylglucose, le distéarate de méthyl glucose et de polyglycérol-3, vendu par la société Goldschmidt sous la dénomination de Tego-care 450®. On peut citer aussi les monoesters de glucose ou de maltose tels que l'o-hexadécanoyle-6-D-glucoside de méthyle et l'o-hexadécanoyle-6-D-maltoside.

Les éthers d'alcool gras et de sucre, utilisables comme tensioactifs dans la composition selon l'invention peuvent être choisis notamment dans le groupe comprenant les éthers ou mélanges d'éthers d'alcool gras en C₈-C₂₂ et de glucose, de maltose, de sucrose ou de fructose et les éthers ou mélanges d'éthers d'alcool gras en C₁₄-C₂₂ et de méthylglucose. Ce sont notamment des alkylpolyglucosides.

Les alcools gras en C₈-C₂₂ ou en C₁₄-C₂₂ formant le motif gras des éthers utilisables selon l'invention comportent une chaîne alkyle linéaire saturée ou non saturée, comportant respectivement de 8 à 22 ou de 14 à 22 atomes de carbone. Le motif gras des éthers peut être notamment choisi parmi les motifs décyle, cétyle, béhényle, arachidyle, stéaryle, palmityle, myristyle, lauryle, capryle, hexadécanoyle et leurs mélanges tels que cétéaryle.

A titre d'exemple d'éthers d'alcool gras et de sucre, on peut citer les alkylpolyglucosides tels que le décyl glucoside et le lauryl glucoside commercialisés par exemple par la société Henkel sous les dénominations respectives Plantaren 2000® et Plantaren 1200®, le cétostéaryl glucoside éventuellement en mélange avec l'alcool cétostéarylique, commercialisé par exemple sous la dénomination Montanov 68® par la société Seppic, sous la dénomination Tego-care CG90® par la société Goldschmidt et sous la dénomination Emulgade KE3302® par la société Henkel, ainsi que l'arachidyl glucoside, par exemple sous la forme du mélange d'alcools arachidique et béhénique et d'arachidyl glucoside commercialisé sous la dénomination Montanov 202® par la société Seppic,
- Les copolymères blocs d'oxyde d'éthylène et d'oxyde de propylène. Les copolymères blocs d'oxyde d'éthylène et d'oxyde de propylène, utilisables comme tensioactifs dans les compositions selon l'invention peuvent être choisis notamment parmi les copolymères blocs de formule (A) :

   HO(C₂H₄O)ₓ(C₃H₆O)_{y}(C₂H₄O)_{z}H (A)

   dans laquelle x, y et z sont des nombres entiers tels que x+z peut aller de 2 à 280 et peut aller de 14 à 100. Ces polymères sont notamment commercialisés sous le nom de Pluronic® ou Lutrol® par BASF, Symperonic® par Uniqema,
- les lécithines de soja ou d'oeuf hydrogénée ou non, enrichies en phosphatidylcholine ou non,
- les tensioactifs siliconé comportant au moins une chaîne oxyéthylénée et ou oxypropylénée. On peut citer, à titre d'exemple, ceux décrits dans les brevets US A 5364633 et US A 5411744 et en particulier un composé de formule (I) : 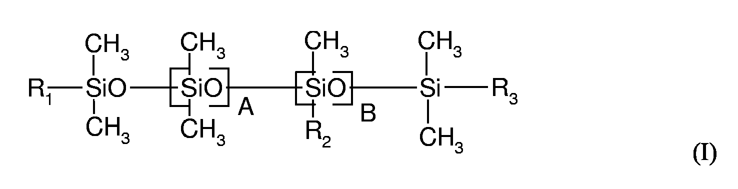
dans laquelle :
R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, au moins un radical R₁, R₂ ou R₃ n'étant pas un radical alkyle ; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation particulier, dans le composé de formule (I), le radical alkyle est un radical méthyle, x est un nombre entier allant de 2 à 6 et y est un nombre entier allant de 4 à 30.

On peut citer, à titre d'exemple de tensioactifs siliconés de formule (I), les composés de formule (II) : 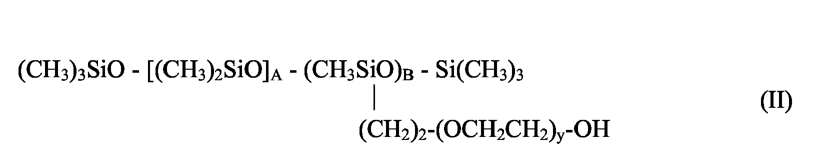 dans laquelle A est un nombre entier allant de 20 à 105, B est un nombre entier allant de 2 à 10 et y est un nombre entier allant de 10 à 20. On peut également citer à titre d'exemple de tensioactifs siliconés de formule (I), les composés de formule (III) :

HO - (CH₂CH₂O)_{y}-(CH₂)₃ - [(CH₃)₂SiO]_{A'} - (CH₂)₃ - (OCH₂CH₂)_{y} - OH (III)

dans laquelle A' et y sont des nombres entiers allant de 10 à 20.

On peut utiliser comme composés de l'invention ceux vendus par la société Dow Corning sous les dénominations DC 5329, DC 7439-146, DC 2-5695 et Q4-3667. Les composés DC 5329, DC 7439-146, DC 2-5695 sont des composés de formule (II) où respectivement A est 22, B est 2 et y est 12 ; A est 103, B est 10 et y est 12 ; A est 27, B est 3 et y est 12. Le composé Q4-3667 est un composé de formule (III) où A est 15 et y est 13. Mais aussi :
- les citrates d'alkyléther,
- les alkényl succinates alkoxylés,
- les alkényl succinates de glucose alkoxylés,
- les alkényl succinates de méthylglucose alkoxylés.

Ces tensioactifs peuvent être utilisés seuls ou en association. Leur taux, par rapport à la phase huileuse encapsulée (après évaporation du solvant) peut être compris entre 0,1 et 30 % en poids.

Afin de parfaire la stabilité de cette émulsion, voire de réduire un peu plus la taille des gouttes du milieu solvant organique, il peut être avantageux d'ajouter de 0,01 à 5 % en poids par rapport au poids total de la dispersion d'au moins un tensioactif ionique, soluble dans l'eau et ayant une HLB supérieure à 11. Ce type de tensioactif ionique génère semble-t-il une charge électrique à la surface des nanocapsules et favorise ainsi la manifestation de répulsions électrostatiques entres elles. Ce ou ces tensioactifs ioniques, anioniques ou cationiques peuvent être choisis parmi :
- les lipides amphiphiles anioniques comme :
   - les sels alcalins du dicétyl- et du dimyristyl-phosphate,
   - les sels alcalins du cholestérol sulfate,
   - les sels alcalins du cholestérol phosphate,
   - les lipoaminoacides et leurs sels tels que les acylglutamates mono- et disodiques comme le sel disodique de l'acide N-stéaroyl L-glutamique commercialisé sous la dénomination Amisoft HS21P par la société AJINOMOTO,
   - les sels de sodium de l'acide phosphatidique,
   - les phospholipides,
   - les dérivés alkylsulfoniques notamment de formule : 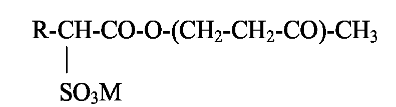
   dans laquelle R représente des radicaux alkyle en C₁₆-C₂₂, en particulier les radicaux C₁₆H₃₃ et C₁₈H₃₇ pris en mélange ou séparément et M est un métal alcalin ou alcalino-terreux tel que le sodium,
- les lipides amphiphiles cationiques de type sels d'ammonium quaternaire, amines grasses et leurs sels comme par exemple :
   - les sels d'ammonium quaternaires de formule générale (IV) suivante : 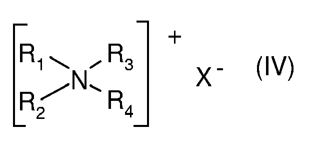 dans laquelle les radicaux R₁, R₂, R₃ et R₄ peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₂-C₆)sulfates, alkyl-ou-alkylarylsulfonates. Comme sels d'ammonium quaternaire de formule (IV), on préfère, d'une part, les chlorures de tétraalkylammonium comme par exemple les chlorures de dialkyldiméthylammonium ou d'alkyltriméthylammonium, dans lesquels le radical alkyle comporte environ de 12 à 22 atomes de carbone, en particulier les chlorures de béhényltriméthylammonium, de distéaryldiméthyl-ammonium, de cétyltriméthyl-ammonium, de benzyl diméthyl stéaryl-ammonium ou encore, d'autre part, le chlorure de stéaramidopropyldiméthyl (acétate de myristyle) ammonium vendu sous la dénomination «CERAPHYL 70®» par la société VAN DYK,
   - les sels d'ammonium quaternaire de l'imidazolinium, comme par exemple ceux de formule (V) suivante : 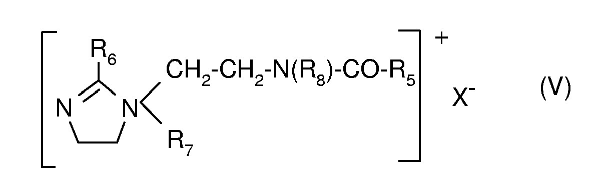 dans laquelle R₅ représente un radical alcényle ou alkyle comportant de 8 à 30 atomes de carbone, par exemple dérivé des acides gras du suif ; R₆ représente un atome d'hydrogène, un radical alkyle comportant de 1 à 4 atomes de carbone ou un radical alcényle ou alkyle comportant de 8 à 30 atomes de carbone ; R₇ représente un radical alkyle comportant de 1 à 4 atomes de carbone ; R₈ représente un atome d'hydrogène, un radical alkyle comportant de 1 à 4 atomes de carbone ; X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone, par exemple dérivés des acides gras du suif, R₇ désigne un radical méthyle, R₈ désigne l'hydrogène. Un tel produit est par exemple vendu sous la dénomination «REWOQUAT W 75» par la société REWO,
   - les sels de diammonium quaternaire de formule (VI) suivante : 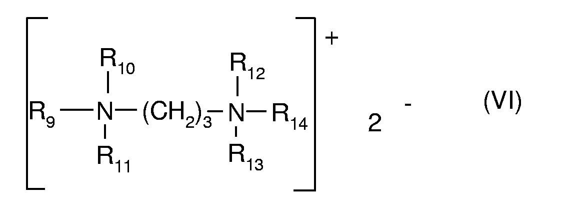
   dans laquelle R₉ désigne un radical aliphatique comportant environ de 16 à 30 atomes de carbone ; R₁₀, R₁₁, R₁₂, R₁₃, et R₁₄ sont choisis parmi l'hydrogène ou un radical alkyle comportant de 1 à 4 atomes de carbone; et X est un anion choisi dans le groupe des halogénures, acétates, phosphates, nitrates et méthylsulfates. De tels sels de diammonium quaternaire comprennent notamment le dichlorure de propane suif diammonium.

Le taux de tensioactif ionique lorsqu'il est associé au(x) tensioactif(s) non ionique(s) présent(s) dans le milieu aqueux est généralement ajusté de manière à représenter de 2 à 100 % en poids du poids de celui-ci.

L'étape c) est réalisée par dispersion du milieu solvant organique (a) dans le milieu aqueux (b). Cette dispersion est réalisée dans des conditions telles que l'on obtienne une pré-émulsion.

Au sens de la présente invention, cette pré-émulsion qualifie un état ou le milieu organique (a) est présent sous la forme de gouttelettes de taille variant de 500 nm à 10 µm, en particulier de 1 à 5 µm au sein du milieu aqueux constituant la phase continue. La taille des gouttelettes peut notamment être contrôlée à l'aide d'un granulomètre laser à diffusion de la lumière.

La formation de la dispersion des nanocapsules attendues est réalisée lors de l'étape (d) en soumettant cette préémulsion à une agitation à effet cisaillant, par exemple à au moins 10000 tr/mn ou à effet de cavitation. Selon un mode de réalisation particulier, cette agitation est obtenue en passant la préémulsion dans un homogénéiseur dit haute pression, généralement sous une pression de 50 à 1500 bars, en particulier de 200 à 1200 bars et notamment supérieur à 400 bars. Cet effet de cisaillement peut également être obtenu à l'aide d'un dispositif ultrasons tel que le Sonitube de la société SODEVA.

Le milieu solvant organique contenu dans cette émulsion est ensuite éliminé, généralement par évaporation selon des techniques classiques, par exemple à l'aide d'un évaporateur rotatif sous pression réduite. Cette évaporation peut être conduite jusqu'à évaporation d'une fraction de la phase aqueuse, indice de l'élimination totale du milieu solvant organique.

A l'issue de cette étape, on obtient une dispersion de nanocapsules conforme à l'invention.

La présente invention a également pour objet l'utilisation d'une dispersion de nanocapsules conforme à l'invention pour la préparation d'une composition cosmétique, dermatologique ou pharmaceutique.

Les nanocapsules selon l'invention peuvent être introduites dans tous les types de formulation galénique liquide, semi-solide, voire solide. Il peut notamment s'agir de gels, d'émulsions huile/eau, eau/huile, multiples (E/H/E, H/E/H), de sérums ou de lotions. On peut par exemple introduire de 0,5 à 60 % d'une dispersion aqueuse de nanocapsules conforme à l'invention dans une composition cosmétique, dermatologique ou pharmaceutique.

Un autre aspect de l'invention concerne une composition cosmétique, dermatologique ou pharmaceutique comprenant dans un milieu physiologiquement acceptable au moins une dispersion aqueuse de nanocapsules selon l'invention.

Les formulations décrites contenant des nanocapsules peuvent notamment être destinées aux soins et/ou au maquillage des cheveux, de la peau et des ongles.

Par ailleurs, les nanocapsules de la présente invention peuvent être utilisées à des fins pharmaceutiques pour la vectorisation de médicament, par voie orale, péritonéale, intra veineuse, intra musculaire ou ophtalmique.

Les exemples figurant ci-après sont présentés à titre illustratif et non limitatif du domaine de l'invention.

L'ensemble des dispersions aqueuses figurant dans les exemples ci-après a été préparé selon le protocole suivant :
Une pré-émulsion de type milieu solvant organique/eau est réalisée à l'aide d'un agitateur type Ultra Turrax (Ika) pendant 5 min à 10000 t/min puis est homogénéisée à l'aide d'un homogénéisateur haute pression type Soavi OBL 20, 2 fois à 500 bars de manière à obtenir la dispersion de nanocapsules attendue. Le solvant est éliminé sous pression réduite à l'aide d'un évaporateur rotatif ainsi qu'une certaine quantité d'eau.

### EXEMPLE 1 : Préparation de nanocapsules d'huile de macadamia

### - Milieu solvant liquide

| | |
|---|---|
| Huile de macadamia | 25,0 g |
| Lécithine de soja | 5,0 g |
| Polyester isophtalique (AQ38S de Eastman Chemical) | 5,0 g |
| Dichlorométhane | 125 ml |

### - Milieu aqueux

| | |
|---|---|
| Eau distillée | 500 ml |
| Poloxamer 338 (commercialisé par BASF) | 2,5 g |

Dans cet exemple, après évaporation du dichlorométhane, on obtient des nanocapsules de 115 nm, parfaitement stables après 2 mois à 45 °C. La dispersion aqueuse finale contient 5 % en poids d'huile et 1 % en poids de polymère, par rapport au poids total de la dispersion.

### Exemple 2 : Préparation de nanocapsules d'huile de graine de coton

### - Milieu solvant organique

| | |
|---|---|
| Huile de graine de coton | 37,5 g |
| Belsil PMS MK (Wacker) | 3,0 g |
| Diméthicone copolyol (DC 2-5695 de Dow Corning) | 6,0 g |
| Dichlorométhane | 250 ml |

### - Milieu aqueux

| | |
|---|---|
| Eau distillée | 375 ml |
| Acide N Stéaroyl L Glutamique disodique (Amisoft HS21P d'Ajinomoto) | 3,0 g |

Suivant le mode opératoire décrit ci-dessus, on obtient une dispersion de nanocapsules possédant une taille moyenne de 98 nm dispersée dans 300 ml d'eau. La dispersion aqueuse contient 12,5 % en poids d'huile de coton et 1 % en poids de polymère. Ces capsules sont parfaitement stables 2 mois à 45 °C.

### Exemple 3 : Préparation de nanocapsules de Vitamine E

### - Milieu solvant organique

| | |
|---|---|
| Acétate de Vitamine E | 75,0 g |
| Acétobutyrate de cellulose (CAB-381-0.5 Eastman) | 11,25 g |
| Diméthicone Copolyol (DC2-5695 de Dow Coming) | 3,75 g |
| Dichlorométhane | 250 ml |

### - Milieu aqueux

| | |
|---|---|
| Eau distillée | 375 ml |
| Acide N Stéaroyl L Glutamique disodique (Amisoft HS21P d'Ajinomoto) | 1,875 g |

Après évaporation du dichlorométhane, on obtient une dispersion de nanocapsules possédant une taille moyenne de 124 nm et parfaitement stables 2 mois à 45 °C. La dispersion aqueuse contient 20 % en poids d'huile et 3 % en poids de polymère.

### Exemple 4 : Préparation de nanocapsules d'isocétyl palmitate

### - Milieu solvant organique

| | |
|---|---|
| Palmitate d'isocétyle | 56,25 g |
| Poly caprolactone (CAPA6100 de SOLVAY) | 7,5 g |
| Diméthicone Copolyol (DC2-5695 de Dow Corning) | 3,75 g |
| Dichlorométhane | 250 ml |

### - Milieu aqueux

| | |
|---|---|
| Eau distillée | 375 ml |
| Acide N Stearoyl L Glutamique disodique (Amisoft HS21P d'Ajinomoto) | 1,875 g |

Après évaporation du dichlorométhane, on obtient une dispersion de nanocapsules possédant une taille moyenne de 103 nm et parfaitement stables 2 mois à 45 °C. La dispersion aqueuse contient 15 % en poids d'huile et 2 % en poids de polymère.

### Exemple 5 : Préparation de nanocapsules de vitamine E

### - Milieu solvant organique

| | |
|---|---|
| Acétate de vitamine E | 75,0 g |
| Eastar Bio® de Eastman Chemical (Poly(tetraméthylène Adipate co térephtalate) | 3,75 g |
| Diméthicone Copolyol (DC2-5695 de Dow Coming) | 3,75 g |
| Dichlorométhane | 250 ml |

### - Milieu aqueux

| | |
|---|---|
| Eau distillée | 375 ml |
| Acide N Stéaroyl L Glutamique disodique (Amisoft HS21P d'Ajinomoto) | 1,875 g |

Après évaporation du dichlorométhane, on obtient une dispersion aqueuse de nanocapsules de taille moyenne de 240 nm parfaitement stables après 2 mois à 45 °C. La dispersion aqueuse contient 20 % en poids d'huile et 1 % en poids de polymère.

## Revendications

1. Dispersion aqueuse de nanocapsules de type noyau/écorce, dans lesquelles :
- le noyau comprend au moins une huile,
- l'écorce recouvrant le noyau est de nature polymérique, non réticulée, non hydrosoluble et non soluble dans l'huile dudit noyau, lesdites nanocapsules présentant une taille moyenne inférieure ou égale à 500 nm et possédant un taux d'encapsulation supérieur ou égal à 10 % en poids exprimé en poids de matière(s) encapsulée(s) par rapport au poids total de ladite dispersion.

2. Dispersion aqueuse selon la revendication 1, **caractérisée en ce que** les nanocapsules présentent une taille moyenne inférieure ou égale à 250 nm, voire inférieure ou égale à 150 nm, et plus particulièrement inférieure ou égale à 100 nm.

3. Dispersion aqueuse selon la revendication 1 ou 2, **caractérisée en ce que** le taux d'encapsulation est supérieur ou égal à 12,5 % en poids, voire supérieur ou égal à 15 % en poids, et plus particulièrement supérieur ou égal à 17,5 % en poids par rapport au poids total de la dispersion.

4. Dispersion aqueuse selon la revendication 1, 2 ou 3, **caractérisée en ce que** le polymère de l'écorce des nanocapsules a un poids moléculaire en poids allant de 1 000 à 500 000, et notamment de 1 500 à 100 000.

5. Dispersion aqueuse selon l'une quelconque des revendications 1 à 4, **caractérisée en ce que** le polymère de l'écorce des nanocapsules présente un point de fusion inférieur à 100 °C.

6. Dispersion aqueuse selon l'une quelconque des revendications 1 à 5, **caractérisée en ce que** l'écorce des nanocapsules comprend au moins un polymère choisi parmi :
- les polymères de cyanoacrylate d'alkyle en C₂-C₁₂,
- les poly-L-lactides, les poly-DL-lactides, les polyglycolides et les copolymères correspondants,
- les polycaprolactones,
- les polymères de l'acide 3-hydroxy butyrique,
- les copolymères de chlorure de vinyle et d'acétate de vinyle,
- l'acéto-phtalate de polyvinyle, l'acéto-phtalate de cellulose,
- le copolymère polyvinylpyrrolidone-acétate de vinyle,
- les polyéthylènevinylacétates,
- les copolymères d'acide et d'ester méthacrylique,
- les polyacrylonitriles,
- les polyacrylamides,
- les polyéthylèneglycols,
- les poly-(méthacrylate d'hydroxyalkyle en C₁ à C₄),
- les dérivés de cellulose,
- le polystyrène et ses copolymères,
- les oligomères styrène alkylalcool,
- les terpolymères d'éthylène, d'acétate de vinyle et d'anhydride maléique,
- les polyamides,
- les polyéthylènes,
- les polypropylènes,
- les organopolysiloxanes,
- les poly (alkylène adipate),
- les polyesters polyol,
- les polymères siliconés de polysilsesquioxane,
- les polyesters dendritiques à fonction hydroxyle terminale, et
- leurs mélanges.

7. Dispersion aqueuse selon l'une quelconque des revendications 1 à 6, **caractérisée en ce que** le polymère de l'écorce des nanocapsules est choisi parmi :
- les polycaprolactones,
- l'acéto-phtalate de polyvinyle, l'acéto-phtalate de cellulose,
- les copolymères d'acide et d'ester méthacrylique,
- les dérivés de cellulose,
- le polystyrène et ses copolymères,
- les polyamides,
- les organopolysiloxanes,
- les poly (alkylène adipate), et
- leurs mélanges.

8. Dispersion aqueuse selon l'une quelconque des revendications 1 à 7, **caractérisée en ce que** la ou les huile(s) présente(s) dans le noyau des nanocapsules est liquide à partir d'une température de 40 °C.

9. Dispersion aqueuse selon l'une quelconque des revendications 1 à 8, **caractérisée en ce que** l'huile encapsulée dans le noyau desdits nanocapsules est choisie parmi les huiles végétales riches en insaturations, notamment l'huile de bourrache, les huiles de poisson, les filtres solaires, les vitamines E, F, K, leurs esters, et leur mélanges.

10. Dispersion aqueuse selon l'une quelconque des revendications 1 à 9, **caractérisée en ce que** l'huile encapsulée dans les nanocapsules comprend en outre au moins une matière active liposoluble.

11. Dispersion aqueuse selon la revendication 10, **caractérisée en ce que** ladite matière active liposoluble est choisie parmi les antibiotiques, les antifongiques, les anesthésiques, les analgésiques, les antiseptiques, les antiviraux, les pesticides, les herbicides, et leurs mélanges.

12. Dispersion aqueuse selon la revendication 10 ou 11, **caractérisée en ce que** ladite matière active liposoluble est choisie parmi les vitamines, notamment la vitamine A, la vitamine D, les carotènes, le β-carotène, l'acide salicylique et leurs dérivés.

13. Procédé de préparation d'une dispersion aqueuse de nanocapsules de type noyau/écorce dont le noyau comprend au moins une huile et l'écorce recouvrant le noyau est de nature polymérique non hydrosoluble, non soluble dans la ou les huile(s) dudit noyau, comprenant les étapes consistant à :
a) préparer un milieu solvant organique liquide monophasique comprenant au moins :
- un solvant organique, présentant une température d'ébullition inférieure à 100 °C,
- un polymère soluble dans ledit milieu solvant,
- une huile, contenant le cas échéant au moins une matière active liposoluble, et
- un tensioactif non ionique,
b) préparer un milieu aqueux contenant au moins un tensioactif non ionique et le cas échéant un tensioactif ionique,
c) disperser le milieu organique (a) dans le milieu aqueux (b) de manière à obtenir une pré-émulsion.
d) soumettre la pré-émulsion obtenue en (c) à une homogénéisation de manière à obtenir la formation d'une dispersion aqueuse de nanocapsules de taille moyenne inférieure ou égale à 500 nm,
e) évaporer le solvant organique, la dispersion aqueuse obtenue à l'issue de l'étape e) présente un taux d'encapsulation supérieur ou égal à 10 % en poids exprimé en poids de matière(s) encapsulée(s) par rapport au poids total de ladite dispersion et
f) récupérer ladite dispersion aqueuse de nanocapsules ainsi obtenue.

14. Procédé selon la revendication 13, **caractérisé en ce que** les nanocapsules obtenues à l'étape d) présentent une taille moyenne inférieure ou égale à 250 nm, en particulier inférieure ou égale à 150 nm.

15. Procédé selon la revendication 13 ou 14, **caractérisé en ce que** la dispersion aqueuse obtenue à l'issue de l'étape e) présente un taux d'encapsulation supérieur ou égal à 12,5 % en poids, voire supérieur ou égal à 15 % en poids par rapport au poids total de la dispersion aqueuse.

16. Procédé selon l'une quelconque des revendications 13 à 15, **caractérisé en ce que** le rapport pondéral huile/polymère de l'étape a) est inférieur ou égal à 30/1.

17. Procédé selon l'une quelconque des revendications 13 à 16, **caractérisé en ce que** la proportion en milieu solvant organique utilisé dans l'étape a) est ajustée de manière à constituer de 5 à 70 % en poids du poids total de la pré-émulsion obtenue à l'issue de l'étape c).

18. Procédé selon l'une quelconque des revendications 13 à 17, **caractérisé en ce que** le solvant est choisi parmi l'acétate d'éthyle, l'acétate de butyle, le dichlorométhane, le cyclohexane, l'heptane, le chloro-1-butane, le chloroforme, le formate d'éthyle et leurs mélanges.

19. Procédé selon l'une quelconque des revendications 13 à 18, **caractérisé en ce que** le polymère est tel que défini selon l'une quelconque des revendications 5 à 8.

20. Procédé selon l'une quelconque des revendications 13 à 19, **caractérisé en ce que** l'huile est telle que définie en revendication 8 ou 9.

21. Procédé selon l'une quelconque des revendications 13 à 20, **caractérisé en ce que** la matière active liposoluble est telle que définie en revendication 12 ou 13.

22. Procédé selon l'une quelconque des revendications 13 à 21, **caractérisé en ce que** le tensioactif non ionique des étapes a) et b) est présent en une teneur allant de 0,05 à 25 % en poids, notamment de 1 à 20 % en poids par rapport au poids du milieu solvant organique.

23. Procédé selon l'une quelconque des revendications 13 à 22, **caractérisé en ce que** le tensioactif non ionique des étapes a) et b) est choisi parmi :
- les alkyl ester ou éther de glycérol ou de polyglycérol constitués de 1 à 10 « motif » glycérol et d'au moins une chaîne alkyle (acide pour les esters et alcool pour les éthers) ayant de 12 à 22 atomes de carbone,
- les esters mixtes d'acides gras ou d'alcool gras, d'acide carboxylique et de glycéryl,
- les éthers gras éthoxylés ou esters gras éthoxylés comprenant de 2 à 50 unités d'oxyde d'éthylène et au moins une chaîne alkyle (acide pour les esters et alcool pour les éthers) ayant de 12 à 22 atomes de carbone,
- les esters gras de sorbitan oxyéthylénés ou non. Ils comprennent au moins un motif sorbitan et dans le cas où ils sont oxyéthylénés, de 2 à 50 motifs d'oxyde d'éthylène, et au moins une chaîne alkyle (acide gras) ayant de 12 à 22 atomes de carbone,
- les esters gras de sucre ou éthers gras de sucre,
- les éthers d'alcool gras et de sucre,
- les copolymères blocs d'oxyde d'éthylène et d'oxyde de propylène,
- les lécithines de soja ou d'oeuf hydrogénée ou non, enrichies en phosphatidylcholine ou non,
- les tensioactifs siliconé comportant au moins une chaîne oxyéthylénée et ou oxypropylénée, et
- leurs mélanges.

24. Procédé selon l'une quelconque des revendications 13 à 22, **caractérisé en ce que** le milieu aqueux de l'étape b) comprend au moins tensioactif ionique présent à raison de 2 à 100 % en poids par rapport au poids de tensioactif non ionique.

25. Procédé selon l'une quelconque des revendications 13 à 24, **caractérisé en ce que** ledit tensioactif ionique de l'étape b) est choisi parmi :
- les sels alcalins du dicétyl- et du dimyristylphosphate,
- les sels alcalins du cholestérol sulfate,
- les sels alcalins du cholestérol phosphate,
- les lipoaminoacides et leurs sels,
- les sels de sodium de l'acide phosphatidique,
- les phospholipides,
- les dérivés alkylsulfoniques,
- les sels d'ammonium quaternaires,
- les sels d'ammonium quaternaire de l'imidazolinium,
- les sels de diammonium quaternaire de formule (VI) suivante : 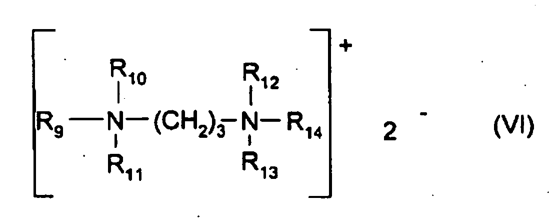
- dans laquelle R9 désigne un radical aliphatique comportant environ de 16 à 30 atomes de carbone ; R₁₀, R₁₁, R₁₂, R₁₃, et R₁₄ sont choisis parmi l'hydrogène ou un radical alkyle comportant de 1 à 4 atomes de carbone ; et X est un anion choisi dans le groupe des halogénures, acétates, phosphates, nitrates et méthylsulfates, et
- leurs mélanges.

26. Procédé selon l'une quelconque des revendications 13 à 25, **caractérisé en ce que** l'homogénéisation est effectuée à l'aide d'un homogénéisateur haute pression, notamment une pression allant de 50 à 1 500 bars, et en particulier allant de 200 à 1 200 bars, ou par ultrasons.

27. Dispersion de nanocapsules obtenue selon le procédé défini selon l'une quelconque des revendications 13 à 26.

28. Utilisation d'une dispersion aqueuse de nanocapsules selon l'une quelconque des revendications 1 à 13 et 27 pour la préparation d'une composition cosmétique, dermatologique ou pharmaceutique.

29. Composition cosmétique, dermatologique ou pharmaceutique comprenant dans un milieu physiologiquement acceptable au moins une dispersion aqueuse telle que définie selon l'une quelconque des revendications 1 à 13 et 27.

## Claims

1. Aqueous dispersion of nanocapsules of core/shell type, in which:
- the core comprises at least one oil,
- the shell covering the core is of non-crosslinked polymer nature and is water-insoluble and insoluble in the oil of the said core,
the said nanocapsules having a mean size of less than or equal to 500 nm and having a rate of encapsulation of greater than or equal to 10 % by weight of encapsulated material relative to the total weight of the said dispersion.

2. Aqueous dispersion according to Claim 1, **characterized in that** the nanocapsules have a mean size of less than or equal to 250 nm, or even less than or equal to 150 nm, and more particularly less than or equal to 100 nm.

3. Aqueous dispersion according to Claim 1 or 2, **characterized in that** the rate of encapsulation is greater than or equal to 12.5 % by weight, or even greater than or equal to 15% by weight, and more particularly greater than or equal to 17.5% by weight relative to the total weight of the dispersion.

4. Aqueous dispersion according to Claim 1, 2 or 3, **characterized in that** the polymer of the shell of the nanocapsules has a weight-average molecular weight ranging from 1000 to 500 000 and especially from 1500 to 100 000.

5. Aqueous dispersion according to any one of Claims 1 to 4, **characterized in that** the polymer of the shell of the nanocapsules has a melting point of less than 100°C.

6. Aqueous dispersion according to any one of Claims 1 to 5, **characterized in that** the shell of the nanocapsules comprises at least one polymer chosen from:
- C₂-C₁₂ alkyl cyanoacrylate polymers,
- poly-L-lactides, poly-DL-lactides, polyglycolides and the corresponding copolymers,
- polycaprolactones,
- 3-hydroxybutyric acid polymers,
- copolymers of vinyl chloride and of vinyl acetate,
- polyvinyl acetophtalate, cellulose acetophtalate,
- poly vinylpyrrolidone/vinyl acetate copolymer,
- poly(ethylene/vinyl acetate)s,
- copolymers of methacrylic acid and ester,
- polyacrylonitriles,
- polyacrylamides,
- polyethylene glycols,
- poly(C₁-C₄ hydroxyalkyl methacrylate)s,
- cellulose derivatives,
- polystyrene and copolymers thereof,
- styrene/alkyl alcohol oligomers,
- terpolymers of ethylene, of vinyl acetate and of maleic anhydride,
- polyamides,
- polyethylenes,
- polypropylenes,
- organopolysiloxanes,
- poly(alkylene adipate)s,
- polyester polyols,
- polysilsesquioxane silicone polymers,
- dendritic polyesters containing a hydroxyl end function, and
- mixtures thereof.

7. Aqueous dispersion according to any one of Claims 1 to 6, **characterized in that** the polymer of the shell of the nanocapsules is chosen from:
- polycaprolactones,
- polyvinyl acetophtalate, cellulose acetophtalate,
- copolymers of methacrylic acid and ester,
- cellulose derivatives,
- polystyrene and copolymers thereof,
- polyamides,
- organopolysiloxanes,
- poly(alkylene adipate)s, and
- mixtures thereof.

8. Aqueous dispersion according to any one of Claims 1 to 7, **characterized in that** the oil(s) present in the core of the nanocapsules is liquid at and above a temperature of 40°C.

9. Aqueous dispersion according to any one of Claims 1 to 8, **characterized in that** the oil encapsulated in the core of the said nanocapsules is chosen from unsaturation-rich plant oils, especially borage oil, fish oils, sunscreens, vitamins E, F and K and esters thereof, and mixtures thereof.

10. Aqueous dispersion according to any one of Claims 1 to 9, **characterized in that** the oil encapsulated in the nanocapsules also comprises at least one liposoluble active material.

11. Aqueous dispersion according to Claim 10, **characterized in that** the said liposoluble active material is chosen from antibiotics, antifungal agents, anaesthetics, analgesics, antiseptic agents, anti-viral agents, pesticides and herbicides, and mixtures thereof.

12. Aqueous dispersion according to Claim 10 or 11, **characterized in that** the said liposoluble active material is chosen from vitamins, especially vitamin A, vitamin D, carotenes, β-carotene and salicylic acid, and derivatives thereof.

13. Process for preparing an aqueous dispersion of nanocapsules of core/shell type, the core of which comprises at least one oil and the shell covering the core is of water-insoluble polymeric nature and is insoluble in the oil(s) of the said core, comprising the steps consisting in:
a) preparing a one-phase liquid organic medium comprising at least:
- one organic solvent, with a boiling point of less than 100°C,
- a polymer that is soluble in the said solvent medium,
- an oil, where appropriate containing at least one liposoluble active material, and
- a nonionic surfactant,
b) preparing an aqueous medium containing at least one nonionic surfactant and, where appropriate, an ionic surfactant,
c) dispersing the organic medium (a) in the aqueous medium (b) so as to obtain a pre-emulsion,
d) subjecting the pre-emulsion obtained in (c) to homogenization so as to obtain the formation of a aqueous dispersion of nanocapsules with a mean size of less than or equal to 500 nm,
e) evaporating off the organic solvent, the aqueous dispersion obtained as a result of e) presents a rate of encapsulation of greater than or equal to 10 % by weight of encapsulated material relative to the total weight of the said dispersion, and
f) recovering the said aqueous dispersion of nanocapsules thus obtained.

14. Process according to Claim 13, **characterized in that** the nanocapsules obtained in step d) are less than or equal to 250 nm and in particular less than or equal to 150 nm in mean size.

15. Process according to Claim 13 or 14, **characterized in that** the aqueous dispersion obtained after step e) has a rate of encapsulation of greater than or equal to 12.5% by weight, or even greater than or equal to 15% by weight, relative to the total weight of the aqueous dispersion.

16. Process according to any one of Claims 13 to 15, **characterized in that** the oil/polymer weight ratio of step a) is less than or equal to 30/1.

17. Process according to any one of Claims 13 to 16, **characterized in that** the proportion of organic solvent medium used in step a) is adjusted so as to constitute from 5% to 70% by weight relative to the total weight of the pre-emulsion obtained after step c).

18. Process according to any one of Claims 13 to 17, **characterized in that** the solvent is chosen from ethyl acetate, butyl acetate, dichloromethane, cyclohexane, heptane, 1-chlorobutane, chloroform and ethyl formate, and mixtures thereof.

19. Process according to any one of Claims 13 to 18, **characterized in that** the polymer is as defined according to any one of Claims 5 to 8.

20. Process according to any one of Claims 13 to 19, **characterized in that** the oil is as defined in Claim 8 or 9.

21. Process according to any one of Claims 13 to 20, **characterized in that** the liposoluble active material is as defined in Claim 12 or 13.

22. Process according to any one of Claims 13 to 21, **characterized in that** the nonionic surfactant of steps a) and b) is present in a content ranging from 0.05% to 25% by weight and especially from 1% to 20% by weight relative to the weight of the organic solvent medium.

23. Process according to any one of Claims 13 to 22, **characterized in that** the nonionic surfactant of steps a) and b) is chosen from:
- alkyl esters or ethers of glycerol or of polyglycerol consisting of from 1 to 10 glycerol "units" and of at least one alkyl chain (acid for the esters and alcohol for the ethers) containing from 12 to 22 carbon atoms,
- mixed esters of fatty acids or of fatty alcohols, of carboxylic acid and of glyceryl,
- ethoxylated fatty ethers or ethoxylated fatty esters comprising from 2 to 50 ethylene oxide units and at least one alkyl chain (acid for the esters and alcohol for the ethers) containing from 12 to 22 carbon atoms,
- oxyethlenated or non-oxyethylenated fatty esters of sorbitan. They comprise at least one sorbitan unit and, when they are oxyethylenated, from 2 to 50 ethylene oxide units, and at least one alkyl chain (fatty acid) containing from 12 to 22 carbon atoms,
- sugar fatty esters or sugar fatty ethers,
- alcohol fatty ethers and sugar ethers,
- block copolymers of ethylene oxide and of propylene oxide,
- hydrogenated or non-hydrogenated soybean or egg lecithins, optionally enriched in phosphatidylcholine,
- silicone surfactants comprising at least one oxyethylene and/or oxypropylene chain, and
- mixtures thereof.

24. Process according to any one of Claims 13 to 22, **characterized in that** the aqueous medium of step b) comprises at least one ionic surfactant present in a proportion of from 2% to 100% by weight relative to the weight of nonionic surfactant.

25. Process according to any one of Claims 13 to 24, **characterized in that** the said ionic surfactant of step b) is chosen from:
- alkali salts of dicetyl and dimyristyl phosphate,
- alkali salts of cholesteryl sulfate,
- alkali salts of cholesteryl phosphate,
- lipoamino acids and salts thereof,
- the sodium salts of phosphatidic acid,
- phospholipids,
- alkylsulfonic derivatives,
- quaternary ammonium salts,
- the quaternary ammonium salts of imidazolinium,
- diquaternary ammonium salts of formula (VI) below: 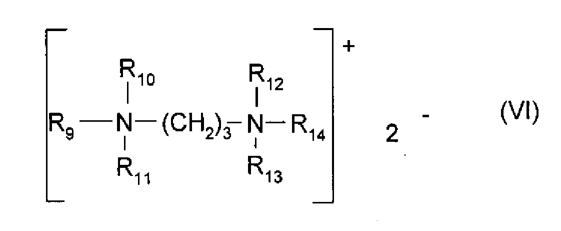 in which R₉ denotes an aliphatic radical containing from about 16 to 30 carbon atoms; R₁₀, R₁₁, R₁₂, R₁₃ and R₁₄ are chosen from hydrogen or an alkyl radical containing from 1 to 4 carbon atoms; and X is an anion chosen from the group of halides, acetates, phosphates, nitrates and methyl sulfates, and
- mixtures thereof.

26. Process according to any one of Claims 13 to 25, **characterized in that** the homogenization is performed using a high-pressure homogenizer, especially at a pressure ranging from 50 to 1500 bar and in particular ranging from 200 to 1200 bar, or by ultrasound.

27. Dispersion of nanocapsules obtained by the process defined according to any one of Claims 13 to 26.

28. Use of an aqueous dispersion of nanocapsules according to any one of Claims 1 to 13 and 27, for the preparation of a cosmetic, dermatological or pharmaceutical composition.

29. Cosmetic, dermatological or pharmaceutical composition comprising, in a physiologically acceptable medium, at least one aqueous dispersion as defined according to any one of Claims 1 to 13 and 27.

## Patentansprüche

1. Wässrige Dispersion von Nanokapseln vom Typ Kern/Schale, in denen:
- der Kern mindestens ein Öl einschließt,
- die Schale, die den Kern umgibt, von polymerer, nicht vernetzter, nicht wasserlöslicher und nicht in dem Öl des Kerns löslicher Natur ist,
wobei die Nanokapseln eine durchschnittliche Größe von kleiner oder gleich 500 nm aufweisen und einen Verkapselungsgehalt von größer oder gleich 10 Gew.-% besitzen, ausgedrückt als Gewicht an verkapselter Substanz (verkapselten Substanzen), bezogen auf das Gesamtgewicht der Dispersion.

2. Wässrige Dispersion nach Anspruch 1, **dadurch gekennzeichnet, dass** die Nanokapseln eine durchschnittliche Größe von kleiner oder gleich 250 nm, sogar von kleiner oder gleich 150 nm und ganz besonders von kleiner oder gleich 100 nm aufweisen.

3. Wässrige Dispersion nach Anspruch 1 oder 2, **dadurch gekennzeichnet, dass** der Verkapselungsgehalt größer oder gleich 12,5 Gew.-%, sogar größer oder gleich 15 Gew.-% und ganz besonders größer oder gleich 17,5 Gew.-%, bezogen auf das Gesamtgewicht der Dispersion, ist.

4. Wässrige Dispersion nach Anspruch 1, 2 oder 3, **dadurch gekennzeichnet, dass** das Polymer der Schale der Nanokapseln ein Molekulargewicht, gewichtbezogen, von 1000 bis 500 000 und insbesondere von 1500 bis 100000 aufweist.

5. Wässrige Dispersion nach einem der Ansprüche 1 bis 4, **dadurch gekennzeichnet, dass** das Polymer der Schale der Nanokapseln einen Schmelzpunkt unterhalb von 100 °C aufweist.

6. Wässrige Dispersion nach einem der Ansprüche 1 bis 5, **dadurch gekennzeichnet, dass** die Schale der Nanokapseln mindestens ein Polymer umfasst, das ausgewählt ist aus:
- C₂-C₁₂-Alkylcyanoacrylatpolymeren,
- Poly-L-lactiden, Poly-DL-lactiden, Polyglycoliden und den entsprechenden Copolymeren,
- Polycaprolactonen,
- 3-Hydroxybuttersäurepolymeren,
- Vinylchlorid- und Vinylacetat-Copolymeren,
- Polyvinylacetophthalat, Celluloseacetophthalat,
- Polyvinylpyrrolidon-Vinylacetat-Copolymeren,
- Polyethylenvinylacetaten,
- Copolymeren von Methacrylsäure und Methacrylsäureester,
- Polyacrylnitrilen,
- Polyacrylamiden,
- Polyethylenglycolen,
- Poly-(C₁-C₄-hydroxyalkylmethacrylat)
- Cellulosederivaten,
- Polystyrol und seinen Copolymeren,
- Styrol-Alkylalkohol-Oligomeren,
- Terpolymeren von Ethylen, Vinylacetat und Maleinsäureanhydrid,
- Polyamiden,
- Polyethylenen,
- Polypropylenen,
- Organopolysiloxanen,
- Poly(alkylenadipat),
- Polyolpolyestern,
- siliconierten Polymeren von Polysilsesquioxan,
- dendritischen Polyestern mit terminaler Hydroxylfunktion, und
- ihren Gemischen.

7. Wässrige Dispersion nach einem der Ansprüche 1 bis 6, **dadurch gekennzeichnet, dass** das Polymer der Schale der Nanokapseln ausgewählt ist aus:
- Polycaprolactonen,
- Polyvinylacetophthalat, Celluloseacetophthalt,
- Copolymeren von Methaccrylsäure und Methacrylsäureester,
- Cellulosederivaten,
- Polystyrol und seinen Copolymeren,
- Polyamiden,
- Organopolysiloxanen
- Poly(alkylenadipat), und
- ihren Gemischen.

8. Wässrige Dispersion nach einem der Ansprüche 1 bis 7, **dadurch gekennzeichnet, dass** das oder die Öle, das (die) in dem Kern der Nanokapseln vorhanden ist (sind), ab einer Temperatur von 40 °C an flüssig sind.

9. Wässrige Dispersion nach einem der Ansprüche 1 bis 8, **dadurch gekennzeichnet, dass** das in dem Kern der Nanokapseln verkapselte Öl ausgewählt ist aus Pflanzenölen, die reich sind an Ungesättigtheiten, insbesondere Borretschöl, Fischölen, Sonnenfiltern, den Vitaminen E, F, K, ihren Estern und ihren Gemischen.

10. Wässrige Dispersion nach einem der Ansprüche 1 bis 9, **dadurch gekennzeichnet, dass** das in den Nanokapseln verkapselte Öl weiterhin mindestens einen fettlöslichen Wirkstoff umfasst.

11. Wässrige Dispersion nach Anspruch 10, **dadurch gekennzeichnet, dass** der fettlösliche Wirkstoff ausgewählt ist aus Antibiotika, Fungiziden, Anästhetika, Analgetika, Antiseptika, antiviralen Mitteln, Pestiziden, Herbiziden und ihren Gemischen.

12. Wässrige Dispersion nach Anspruch 10 oder 11, **dadurch gekennzeichnet, dass** der fettlösliche Wirkstoff ausgewählt ist aus Vitaminen, insbesondere Vitamin A, Vitamin D, Carotinen, β-Carotin, Salicylsäure und ihren Gemischen.

13. Verfahren zur Herstellung einer wässrigen Dispersion von Nanokapseln vom Typ Kern/Schale, deren Kern mindestens ein Öl einschließt und die Schale, die den Kern umgibt, von polymerer, nicht wasserlöslicher und nicht in dem (den) Öl(en) des Kerns löslicher Natur ist, das die Schritte einschließt, die bestehen aus:
a) Herstellen eines flüssigen, einphasigen, organischen Lösungsmittelmediums, das mindestens folgendes einschließt:
- ein organisches Lösungsmittel, das eine Siedetemperatur von kleiner als 100 °C aufweist,
- ein in dem Lösungsmittelmedium lösliches Polymer,
- ein Öl, das gegebenenfalls mindestens einen fettlöslichen Wirkstoff enthält, und
- ein nichtionisches Tensid,
b) Herstellen eines wässrigen Mediums, das mindestens ein nichtionisches Tensid und gegebenenfalls ein ionisches Tensid enthält,
c) Dispergieren des organischen Mediums (a) in dem wässrigen Medium (b) derart, dass eine Voremulsion erhalten wird,
d) Durchführen einer Homogenisation mit der unter (c) erhaltenen Voremulsion derart, dass die Bildung einer wässrigen Dispersion von Nanokapseln einer durchschnittlichen Größe von kleiner oder gleich 500 nm erhalten wird,
e) Verdampfen des organischen Lösungsmittels, die am Ende von Schritt e) erhaltene wässrige Dispersion weist einen Verkapselungsgehalt von größer oder gleich 10 Gew.-% auf, ausgedrückt in Gew.-% der verkapselten Substanz bezogen auf das Gesamtgewicht der Dispersion, und
f) Gewinnen der so erhaltenen wässrigen Dispersion von Nanokapseln.

14. Verfahren nach Anspruch 13, **dadurch gekennzeichnet, dass** die in Schritt d) erhaltenen Nanokapseln eine durchschnittliche Größe von kleiner oder gleich 250 nm, insbesondere von kleiner oder gleich 150 nm aufweisen.

15. Verfahren nach Anspruch 13 oder 14, **dadurch gekennzeichnet, dass** die am Ende von Schritt e) erhaltene wässrige Dispersion einen Verkapselungsgehalt von größer oder gleich 12,5 Gew.-%, sogar von größer oder gleich 15 Gew.-%, bezogen auf das Gesamtgewicht der wässrigen Dispersion, aufweist.

16. Verfahren nach einem der Ansprüche 13 bis 15, **dadurch gekennzeichnet, dass** das Gewichtsverhältnis Öl/Polymer von Schritt a) kleiner oder gleich 30/1 ist.

17. Verfahren nach einem der Ansprüche 13 bis 16, **dadurch gekennzeichnet, dass** der Anteil an in dem Schritt a) verwendetem organischem Lösungsmittelmedium derart eingestellt wird, dass er 5 bis 70 Gew.-% des Gesamtgewichts der am Ende von Schritt c) erhaltenen Voremulsion ausmacht.

18. Verfahren nach einem der Ansprüche 13 bis 17, **dadurch gekennzeichnet, dass** das Lösungsmittel ausgewählt ist aus Ethylacetat, Butylacetat, Dichlormethan, Cyclohexan, Chlor-1-butan, Chloroform, Ethylformiat und ihren Gemischen.

19. Verfahren nach einem der Ansprüche 13 bis 18, **dadurch gekennzeichnet, dass** das Polymer so ist, wie nach einem der Ansprüche 5 bis 8 definiert.

20. Verfahren nach einem der Ansprüche 13 bis 19, **dadurch gekennzeichnet, dass** das Öl so ist, wie in Anspruch 8 oder 9 definiert.

21. Verfahren nach einem der Ansprüche 13 bis 20, **dadurch gekennzeichnet, dass** der fettlösliche Wirkstoff so ist, wie in Anspruch 12 oder 13 definiert.

22. Verfahren nach einem der Ansprüche 13 bis 21, **dadurch gekennzeichnet, dass** das nichtionische Tensid der Schritte a) und b) in einem Gehalt von 0,05 bis 25 Gew.-%, insbesondere von 1 bis 20 Gew.-%, bezogen auf das Gewicht des organischen Lösungsmittelmediums, vorhanden ist.

23. Verfahren nach einem der Ansprüche 13 bis 22, **dadurch gekennzeichnet, dass** das nichtionische Tensid der Schritte a) und b) ausgewählt ist aus:
- Glycerin- oder Polyglycerinalkylester oder -ether, bestehend aus 1 bis 10 "Glycerin-"Einheiten" und mindestens einer Alkylkette (Säure für die Ester und Alkohol für die Ether) mit 12 bis 22 Kohlenstoffatomen,
- Mischestern von Fettsäuren oder Fettalkoholen, von Carboxylsäure und von Glyceryl,
- ethoxylierten Fettsäureethern oder ethoxylierten Fettsäureestern, die 2 bis 50 Ethylenoxid-Einheiten und mindestens eine Alkylkette (Säure für die Ester und Alkohol für die Ether) mit 12 bis 22 Kohlenstoffatomen einschließen,
- oxyethylenierten oder nicht oxyethylenierten Sorbitanfettsäureestern. Sie umfassen mindestens eine Sorbitan-Einheit und in dem Fall, in dem sie oxyethyleniert sind, 2 bis 50 Ethylenoxid-Einheiten und mindestens eine Alkylkette (Fettsäure) mit 12 bis 22 Kohlenstoffatomen,
- Fettsäureestern von Zucker oder Fettsäureethern von Zucker,
- Ethern von Fettalkohol und Zucker,
- Blockcopolymeren von Ethylenoxid und von Propylenoxid,
- hydrierten oder nicht hydrierten Lecitinen von Soja oder Ei, angereichert oder nicht angereichert an Phosphatidylcholin,
- siliconierten Tensiden, die mindestens eine oxyethylenierte und/oder eine o-xypropylenierte Kette umfassen, und
- ihren Gemischen.

24. Verfahren nach einem der Ansprüche 13 bis 22, **dadurch gekennzeichnet, dass** das wässrige Medium von Schritt b) mindestens ein ionisches Tensid einschließt, das in einem Verhältnis von 2 bis 100 Gew.-%, bezogen auf das Gewicht von nichtionischem Tensid, vorhanden ist.

25. Verfahren nach einem der Ansprüche 13 bis 24, **dadurch gekennzeichnet, dass** das ionische Tensid von Schritt b) ausgewählt ist aus:
- alkalischen Salzen von Dicetyl- und Dimyristylphosphat,
- alkalischen Salzen von Cholesterinsulfat,
- alkalischen Salzen von Cholesterinphosphat,
- Lipoaminosäuren und ihren Salzen,
- Natriumsalzen der Phosphatidsäure,
- Phospholipiden,
- Alkylsulfonsäurederviaten,
- quaternären Ammoniumsalzen,
- quaternären Imidazoliniumammoniumsalzen,
- quaternären Diammoniumsalzen der folgenden Formel (VI): 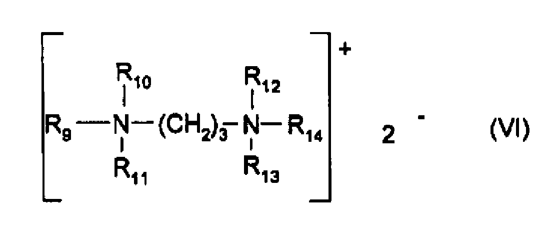
- worin R9 einen aliphatischen Rest bezeichnet, der etwa 16 bis 30 Kohlenstoffatome umfasst; R₁₀, R₁₁, R₁₂, R₁₃ und R₁₄ ausgewählt sind aus Wasserstoff oder einem Alkylrest, der 1 bis 4 Kohlenstoffatome einschließt; und X ein Anion ist, das ausgewählt ist aus der Gruppe von Halogenen, Acetaten, Phosphaten, Nitraten und Methylsulfaten, und
- ihren Gemischen.

26. Verfahren nach einem der Ansprüche 13 bis 25, **dadurch gekennzeichnet, dass** die Homogenisation mit Hilfe eines Hochdruckhomogenisators, insbesondere ein Druck von 50 bis 1500 bar und speziell ein Druck von 200 bis 1200 bar, oder durch Ultraschall durchgeführt wird.

27. Dispersion von Nanokapseln, die durch das Verfahren nach einem der Ansprüche 13 bis 26 erhalten wird.

28. Verwendung einer wässrigen Dispersion von Nanokapseln nach einem der Ansprüche 1 bis 13 und 27 zur Herstellung einer kosmetischen, dermatologischen oder pharmazeutischen Zusammensetzung.

29. Kosmetische, dermatologische oder pharmazeutische Zusammensetzung, die in einem physiologisch verträglichen Medium mindestens eine wässrige Dispersion wie nach einem der Ansprüche 1 bis 13 und 27 definiert umfasst.
